# EUROPEAN PATENT APPLICATION

(11) **EP 3 473 234 A1**
(43) Date of publication of application: **24.04.2019**
(21) Application number: 17813236.1
(22) Date of filing: 09.06.2017
(51) Int. Cl.: A61K 8/22, A61K 8/73, A61K 8/99, A61Q 5/10

(54) **POWDER HAIR DYE COMPOSITION**

(30) Priority: 15.06.2016 JP 2016119256
(71) Applicant: Hoyu Co., Ltd., Nagoya-shi, Aichi 461-8650 (JP)
(72) Inventor: FUTATSUGI Shizuka, Nagakute-shi Aichi 480-1136 (JP); UESAWA Yoshiyuki, Nagakute-shi Aichi 480-1136 (JP); MATSUBAYASHI Jun, Nagakute-shi Aichi 480-1136 (JP); UMINO Hiromi, Nagakute-shi Aichi 480-1136 (JP)
(74) Representative: Grünecker Patent- und Rechtsanwälte PartG mbB
(86) International application number: PCT/JP2017/021520
(87) International publication number: WO 2017/217339

(57) **Abstract**

The purpose of the present invention is, in powder hair dye compositions that contain a percarbonate, to suppress deterioration of coatability in long-term storage and also to ameliorate hair stiffening after hair dyeing treatment. To solve the aforementioned problem, a powder hair dye composition containing (A) a percarbonate and (B) a water-soluble polymer compound is provided, characterized by containing, as the water-soluble polymer compound (B), microorganism-derived/produced mucilage or chitosan containing (b1) glucose as a constitutive sugar, and (b2) a starch. With this powder hair dye composition, it is possible to suppress reduction in coatability in long-term storage under high-temperature, high-humidity conditions, and also to ameliorate hair stiffness after hair dyeing treatment.

## Description

### TECHNICAL FIELD

The present invention relates to a powder hair dye composition for dyeing hair and the like. More specifically, the invention relates to a powder hair dye composition of which the coating operability is not impaired even when being stored under severe conditions such as a high temperature and a high humidity and which has excellent storage stability by using a specific water-soluble polymer compound.

### BACKGROUND ART

Hair dye compositions are those for dyeing hair by oxidizing an oxidation dye and thus developing a color on the hair. As hair dye compositions, a powder hair dye composition which is in a powder form is known in addition to those that are in a liquid form and a cream form. This powder hair dye composition is used by being mixed with a liquid medium such as water to be prepared into a hair dye coating liquid and then applied to the hair.

A water-soluble polymer compound is blended in a powder hair dye composition in order to enhance the coating operability of the hair dye coating liquid. This makes it possible to increase the viscosity of the hair dye coating liquid and thus to improve ease of handling the hair dye coating liquid with a brush, spreading and adhesive property (coating operability) of the hair dye coating liquid to the hair. However, in powder hair dye compositions that contain a percarbonate, there has been a problem that the action of increasing the viscosity by the water-soluble polymer compound is diminished and thus the coating operability is diminished by long-term storage. In order to solve this problem, powder hair dye compositions that contain various polymer compounds have been developed.

For example, Patent Document 1 discloses that the mixing property with a liquid medium such as water and temporary stability of coating operability in long-term storage are improved by concurrently using carboxymethyl cellulose and a water-soluble polymer compound such as xanthan gum in powder hair dye compositions that contain a percarbonate.

### CITATION LIST

### PATENT DOCUMENT

Patent Document 1: WO 2015/052757 A

### SUMMARY OF THE INVENTION

### PROBLEM TO BE SOLVED BY THE INVENTION

Powder hair dye compositions are demanded to have further improved storage stability since there is a case in which the powder hair dye compositions are stored for a long period of time under high-temperature and high-humidity conditions.

In addition, powder hair dye compositions have a problem that the hair feels stiff after a hair dyeing treatment.

In view of the above, an object of the invention is to suppress deterioration in the coating operability in long-term storage under high-temperature and high-humidity conditions and also to ameliorate the hair stiffness after a hair dyeing treatment in powder hair dye compositions that contain a percarbonate and a water-soluble polymer compound.

### MEANS FOR SOLVING PROBLEM

As a result of intensive investigations on the above-mentioned problem, the inventors have found out that it is possible to suppress deterioration in the coating operability in long-term storage under high-temperature and high-humidity conditions and also to ameliorate the hair stiffness after a hair dyeing treatment by using a specific water-soluble polymer compound and thus have completed the invention.

In other words, the invention is the following powder hair dye composition and a method of using the same.

The powder hair dye composition of the invention is a powder hair dye composition which contains (A) a percarbonate and (B) a water-soluble polymer compound and in which (B) the water-soluble polymer compound contains (b1) a microorganism-derived/produced mucilage or chitosan containing glucose as a constitutive sugar and (b2) a starch.

According to this powder hair dye composition, it is possible to suppress deterioration in the coating operability in long-term storage under high-temperature and high-humidity conditions and further to ameliorate the hair stiffness after a hair dyeing treatment.

In addition, according to an embodiment of the powder hair dye composition of the invention, a mass ratio (b2/b1) of (b2) the starch to (b1) the microorganism-derived/produced mucilage or chitosan containing glucose as a constitutive sugar is from 0.1 to 5.

According to this feature, it is possible to further exert the effect of the invention that deterioration is suppressed in the coating operability in long-term storage under high-temperature and high-humidity conditions and also the hair stiffness after a hair dyeing treatment is ameliorated.

Furthermore, according to an embodiment of the powder hair dye composition of the invention, the powder hair dye composition contains (C) a metal salt of stearic acid at from 0.1 to 2 mass%.

According to this feature, it is possible to provide a powder hair dye composition which has excellent mixing property with a liquid medium such as water and also excellent fluidity.

Furthermore, according to an embodiment of the powder hair dye composition of the invention, the powder hair dye composition contains (D) a chelating agent at from 1 to 5 mass%.

According to this feature, it is possible to exert an effect of not only improving the stability of the oxidizing agent but also further ameliorating the hair stiffness after a hair dyeing treatment.

Furthermore, according to an embodiment of the powder hair dye composition of the invention, (b1) is xanthan gum, a content of the xanthan gum is from 8 to 18 mass% and a content of (b2) the starch is from 7 to 35 mass%, and a mass ratio (b2/D) of (b2) the starch to (D) the chelating agent is 3.5 or more.

According to this feature, it is possible to exert an effect of suppressing deterioration in the coating operability in long-term storage under high-temperature and high-humidity conditions, an effect of ameliorating the hair stiffness after a hair dyeing treatment, and an effect of having excellent levelness of dyeing in a well-balanced manner.

The method of using a powder hair dye composition of the invention includes a step of mixing the powder hair dye composition of the invention with a liquid medium to prepare a hair dye coating liquid and a step of applying the hair dye coating liquid to hair.

According to this method of using a powder hair dye composition, it is possible to prepare a hair dye coating liquid having excellent coating operability. In addition, it is possible to provide a method of hair dyeing treatment by which excellent levelness of dyeing is exerted and the hair stiffness at the time of finishing is diminished.

### EFFECT OF THE INVENTION

According to the invention, it is possible to provide a powder hair dye composition in which deterioration is suppressed in the coating operability in long-term storage and further the hair stiffness after a hair dyeing treatment is ameliorated in powder hair dye compositions that contain a percarbonate.

### MODE(S) FOR CARRYING OUT THE INVENTION

Next, the invention will be described including the best modes for carrying out the invention.

### [Powder hair dye composition]

The powder hair dye composition of the invention is a powder hair dye composition which contains (A) a percarbonate and (B) a water-soluble polymer compound and in which (B) the water-soluble polymer compound contains (b1) a microorganism-derived/produced mucilage or chitosan containing glucose as a constitutive sugar and (b2) a starch.

The powder hair dye composition of the invention is used for the purpose of dyeing the hair and is an oxidation hair dye which contains at least an oxidizing agent and an oxidation dye. When the oxidation hair dye acts on the hair, the hair is dyed in the desired color tone by the action of the oxidizing agent. A direct dye may be blended in the oxidation hair dye for adjustment of the color tone of dyed hair.

The powder hair dye composition of the invention is a powder preparation of a hair dye composition, and the respective components such as an oxidizing agent and an oxidation dye are also blended in a powder form. The powder hair dye composition is usually a one-component type but may be a multi-component type of a two- or more-component type. The powder hair dye composition is applied to the hair by being mixed with a liquid medium such as water and thus prepared into a hair dye coating liquid at the time of use.

Next, the respective components to be used in the powder hair dye composition of the invention will be described in detail. Incidentally, the contents of the respective components indicate the contents of the respective agents in the mixture before being mixed with the liquid medium in the case of preparing the powder hair dye composition into a multi-component type.

### <(A) Percarbonate>

A percarbonate is an oxidizing agent and has an action of oxidizing the oxidation dye to develop a color and an action of decomposing melanin inside the hair. Specific examples thereof may include sodium percarbonate and potassium percarbonate. Among these, sodium percarbonate is preferable.

The content of percarbonate in the powder hair dye composition is not particularly limited, but it is, for example, from 15 to 60 mass%, and the lower limit thereof is preferably 20 mass% or more and more preferably 25 mass% or more, and the upper limit thereof is preferably 50 mass% or less and more preferably 40 mass% or less. When the content of percarbonate is 15 mass% or more, an effect is exerted that the hair dyeing power is improved and further the levelness of dyeing is excellent. In addition, when the content of percarbonate is 50 mass% or less, an effect is exerted that the hair is hardly damaged.

The powder hair dye composition of the invention may contain an optional oxidizing agent in addition to (A) the percarbonate. Examples of the optional oxidizing agent may include various kinds of peroxide salts, hydrogen peroxide adducts of various kinds of sulfates, hydrogen peroxide adducts of various kinds of phosphates, hydrogen peroxide adducts of various kinds of pyrophosphates, urea peroxide, melamine peroxide, various kinds of perborates, various kinds of perbromates, and various kinds of permanganates.

In the case of concurrently using (A) the percarbonate and an optional oxidizing agent, the content of the optional oxidizing agent is preferably 5 mass% or less from the viewpoint of imparting a deep color tone to the hair after finishing. In addition, the total content of the oxidizing agents is preferably 60 mass% or less from the viewpoint of improving the temporal stability of the oxidizing agents in the case of being stored for a long period of time.

### <(B) Water-soluble polymer compound>

The water-soluble polymer compound adjusts the viscosity of the hair dye coating liquid, and it is possible to improve ease of handling the hair dye coating liquid with a brush, spreading and adhesive property of the hair dye coating liquid to the hair and thus to enhance the coating operability by containing this. The powder hair dye composition of the invention contains (b1) a microorganism-derived/produced mucilage or chitosan containing glucose as a constitutive sugar and (b2) a starch as the water-soluble polymer compound.

### [(b1) microorganism-derived/produced mucilage or chitosan containing glucose as constitutive sugar]

The microorganism-derived/produced mucilage or chitosan containing glucose as a constitutive sugar (hereinafter referred to as the "produced mucilage") is a natural polymer containing glucose as a constitutive sugar and is produced through the fermentation by microorganisms or an enzyme reaction derived from microorganisms. The fermentation by microorganisms or an enzyme reaction derived from microorganisms may be a reaction by which the produced mucilage is synthesized from glucose in addition to a reaction by which polysaccharides such as starch are decomposed. Specific examples of the produced mucilage may include curdlan, xanthan gum, gellan gum, cyclodextrin, dextran, pullulan, and succinoglucan.

In addition, chitosan is obtained by deacetylating chitin to be obtained from the exoskeleton of crustaceans such as crabs and shrimp through a boiling treatment or the like in a concentrated alkali.

As the component b1 in the invention, xanthan gum and chitosan are preferable and xanthan gum is particularly preferable.

In addition, the substance selected from the produced mucilage or chitosan may be used singly or in combination of two or more kinds thereof.

The content of the component (b1) in the powder hair dye composition is preferably from 1 to 40 mass%. The lower limit value thereof is more preferably 3 mass% or more, still more preferably 5 mass% or more, and particularly preferably 8 mass% or more. The upper limit value thereof is more preferably 30 mass% or less, still more preferably 25 mass% or less, and particularly preferably 18 mass% or less.

By concurrently using the component b1 with the component b2, it is possible to suppress deterioration in the coating operability in long-term storage and further to ameliorate the hair stiffness after a hair dyeing treatment.

In addition, the effect is exerted that the hair stiffness after a hair dyeing treatment is ameliorated even in the case of adding only the component b1.

### [(b2) Starch]

A starch is a water-soluble polymer compound containing amylose and amylopectin as a main component and also includes modified starches which have been physically or chemically modified to have improved functional properties in addition to natural starches. Specific examples thereof may include rice starch, potato starch, sweet potato starch, corn starch, tapioca starch, and wheat starch. Potato starch, rice starch, and corn starch are preferable and potato starch is more preferable. These can be used singly or in combination of two or more kinds thereof.

The content of the component b2 in the powder hair dye composition is preferably from 3 to 50 mass%. The lower limit value thereof is more preferably 6 mass% or more, still more preferably 7 mass% or more, and particularly preferably 10 mass% or more. The upper limit value thereof is more preferably 40 mass% or less, still more preferably 35 mass% or less, and particularly preferably 30 mass% or less.

By concurrently using the component b2 with the component b1, it is possible to suppress deterioration in the coating operability in long-term storage and further to ameliorate the hair stiffness after a hair dyeing treatment. In addition, the effect is exerted that the levelness of dyeing is improved by containing the component b2.

In addition, the mass ratio (b2/b1) of the component b2 to the component b1 is not particularly limited, but it is preferably from 0.1 to 5 from the viewpoint of further exerting the effect of the invention that deterioration is suppressed in the coating operability in long-term storage and also the hair stiffness after a hair dyeing treatment is ameliorated. The lower limit value thereof is more preferably 0.7 or more and particularly preferably 1.5 or more. The upper limit value thereof is more preferably 4.5 or less and particularly preferably 4 or less.

### [Other water-soluble polymer compounds]

A water-soluble polymer compound other than the component b1 and component b2 described above may be blended in the powder hair dye composition of the invention.

Specific examples of other water-soluble polymer compounds may include a natural polymer, a semisynthetic polymer, a synthetic polymer, and an inorganic polymer other than the component b1 and the component b2.

Examples of the natural polymer other than the component b1 and the component b2 may include seaweed extracts such as alginic acid, carrageenan, agar, and furcellaran, seed mucilage such as guar gum, quince seed, konjak mannan, tamarind gum, tara gum, dextrin, and locust bean gum, sap mucilage such as gum arabic, gum ghatti, gum karaya, and gum tragacanth, fruit mucilage such as arabinogalactan, pectin, and quince, plant-based proteins such as wheat protein and soybean protein, animal-based proteins such as albumin, casein, gelatin, and collagen, and mucopolysaccharides such as hyaluronic acid.

Examples of the semisynthetic water-soluble polymer compound may include methyl cellulose, ethyl cellulose, hydroxyethyl cellulose, hydroxypropyl cellulose, carboxymethyl cellulose, sodium carboxymethyl cellulose, methyl hydroxypropyl cellulose, cationized cellulose, alginic acid propylene glycol ester, and an alginate (for example, sodium alginate).

Examples of the synthetic water-soluble polymer compound may include polyvinyl alcohol, polyvinyl pyrrolidone, polyvinyl methyl ether, carboxyvinyl polymer, sodium polyacrylate, polyacrylamide, polyethylene oxide, an ethylene oxide-propylene oxide block copolymer, and an acrylic acid-alkyl acrylate copolymer. In addition to these, examples thereof may also include a copolymer composed of a half ester of itaconic acid with polyoxyethylene alkyl ether or an ester of methacrylic acid with polyoxyethylene alkyl ether and at least one monomer selected from acrylic acid, methacrylic acid, or an alkyl ester thereof.

### <(C) Metal salt of stearic acid>

It is preferable that the powder hair dye composition of the invention contains (C) a metal salt of stearic acid. Specific examples of the metal salt of stearic acid may include magnesium stearate and calcium stearate, and magnesium stearate is preferable among these.

The content of the metal salt of stearic acid in the powder hair dye composition is preferably from 0.1 to 2 mass%. The lower limit value thereof is more preferably 0.5 mass% or more, still more preferably 0.7 mass% or more, and particularly preferably 0.9 mass% or more. The upper limit value thereof is more preferably 2 mass% or less, still more preferably 1.5 mass% or less, and particularly preferably 1.3 mass% or less. In a case in which the content of the metal salt of stearic acid is in the above range, the powder hair dye composition can have excellent mixing property with a liquid medium such as water and the fluidity of the powder hair dye composition can also be improved.

### <(D) Chelating agent>

It is preferable that the powder hair dye composition of the invention contains (D) a chelating agent. The chelating agent is a substance which captures a metal ion, and examples thereof may include L-aspartic acid N,N-diacetic acid tetrasodium salt, alanine, ethylenediamine hydroxyethyl triacetic acid trisodium salt, edetic acid, disodium edetate, edetate calcium disodium, trisodium edetate, tetrasodium edetate, sodium citrate, gluconic acid, L-glutamic acid diacetic acid tetrasodium salt, tartaric acid, phytic acid, sodium polyphosphate, and sodium metaphosphate, and ethylenediamine hydroxyethyl triacetic acid trisodium salt, disodium edetate, and tetrasodium edetate are preferable and ethylenediamine hydroxyethyl triacetic acid trisodium salt is more preferable.

By containing a chelating agent, it is possible to not only enhance the stability of the oxidizing agent but also to exert the effect of ameliorating the hair stiffness of the hair at the time of finishing.

The content of chelating agent in the powder hair dye composition is preferably from 1 to 5 mass%. The lower limit value thereof is more preferably 1.2 mass% or more, still more preferably 1.5 mass% or more, and particularly preferably 1.7 mass% or more. The upper limit value thereof is more preferably 4.5 mass% or less, still more preferably 4 mass% or less, and particularly preferably 3 mass% or less. In a case in which the content of chelating agent is in the above range, it is possible to further exert the effect of stabilizing the oxidizing agent and the effect of ameliorating the hair stiffness at the time of finishing.

In addition, the mass ratio (b2/D) of (b2) the starch to (D) the chelating agent in the powder hair dye composition of the invention is not particularly limited, but it is preferably 3.5 or more, more preferably 5 or more, still more preferably 7 or more, and particularly preferably 10 or more.

By adjusting the mass ratio of (b2) the starch to (D) the chelating agent to be in the above range, it is possible to exert the effect of suppressing deterioration in the coating operability in long-term storage under high-temperature and high-humidity conditions, the effect of ameliorating the hair stiffness after a hair dyeing treatment, and the effect of having excellent levelness of dyeing.

### <Oxidation dye>

The oxidation dye is a dye which is oxidatively polymerized by (A) the percarbonate and develops a color.

The oxidation dye includes a dye intermediate and a coupler, and the dye intermediate is a substance which develops a color by the oxidation of itself and the coupler is a substance to exhibit various color tones in combination with dye intermediates.

The dye intermediate is a dye precursor which is mainly an o- or p-phenylenediamine or an aminophenol and itself is usually a colorless or weakly colored compound.

Specific examples thereof may include p-aminophenol, o-aminophenol, p-methylaminophenol, p-phenylenediamine, toluene-2,5-diamine, N-phenyl-p-phenylenediamine, 4,4'-diaminodiphenylamine, 2-hydroxyethyl-p-phenylenediamine, o-chloro-p-phenylenediamine, 4-amino-m-cresol, 2-amino-4-hydroxyethylaminoanisole, 2,4-diaminophenol, 2,2'-[(4-aminophenyl)imino]bisethanol, and salts such as sulfates and hydrochlorides thereof.

As the kind of dye intermediate, one kind or two or more kinds can be selected and used depending on the desired color tone of the hair. In addition, the content thereof is not particularly limited, but it is, for example, from 1 to 30 mass% in the powder hair dye composition, the lower limit value thereof is more preferably 5 mass% or more, and the upper limit value thereof is more preferably 20 mass% or less.

Examples of the coupler may mainly include m-diamines, m-aminophenols, or m-diphenols, and specific examples thereof may include m-aminophenol, 5-amino-o-cresol, resorcin, catechol, pyrogallol, phloroglucin, gallic acid, hydroquinone, 5-(2-hydroxyethylamino)-2-methylphenol, m-phenylenediamine, 2,4-diaminophenoxyethanol, toluene-3,4-diamine, α-naphthol, 2,6-diaminopyridine, diphenylamine, 3,3'-iminodiphenyl, 1,5-dihydroxynaphthalene, tannic acid, 1-hydroxyethyl-4,5-diamino pyrazole, and salts such as sulfates and hydrochlorides thereof.

As the kind of coupler, one kind or two or more kinds can be selected and used depending on the desired color tone of the hair. In addition, the content thereof is not particularly limited, but it is, for example, from 0.1 to 20 mass% in the powder hair dye composition, the lower limit value thereof is more preferably 0.5% by mass or more and particularly preferably 1% by mass or more. The upper limit value thereof is more preferably 15 mass% or less and particularly preferably 10 mass% or less.

### <Other components>

The powder hair dye composition of the invention may contain optional components in addition to the components described above, if necessary.

Examples of the other components may include an alkali agent, a direct dye, an oil component, a surfactant, an inorganic salt, a dispersant, a pH adjusting agent, a saccharide, a hair growth component, a plant extract, a herbal medicine extract, an amino acid-polypeptide, vitamins, a perfume, a preservative, and an ultraviolet absorber.

### <Alkali agent>

The alkali agent has an action of expanding the hair and thus promoting permeation of the dye and oxidizing agent into the hair. Examples of the alkali agent may include a silicate, a carbonate, a hydrogencarbonate, a metasilicate, a phosphate, a basic amino acid, and a hydroxide. Specific examples of the silicate may include sodium silicate and potassium silicate, examples of the carbonate may include sodium carbonate, ammonium carbonate, magnesium carbonate, and guanidine carbonate, examples of the hydrogencarbonate may include sodium hydrogencarbonate, and ammonium hydrogencarbonate, examples of the metasilicate may include sodium metasilicate and potassium metasilicate, examples of the phosphate may include primary ammonium phosphate, secondary ammonium phosphate, disodium hydrogenphosphate, and trisodium phosphate, examples of the basic amino acid may include arginine, lysine, and a salt thereof, and examples of the hydroxide may include calcium hydroxide and magnesium hydroxide.

### <Direct dye>

The direct dye is a compound exhibiting a color and is a dye which adheres to or permeates the hair to dye the hair. Examples thereof may include an acidic dye, a basic dye, a natural dye, a nitro dye, a HC dye, and a disperse dye. These direct dyes may be blended singly or in combination.

Examples of the acidic dye may include Red No. 2, Red No. 3, Red No. 102, Red No. 104 (1), Red No. 105 (1), Red No. 106, Red No. 227, Red No. 230 (1), Yellow No. 4, Yellow No. 5, Yellow No. 202 (1), Yellow No. 202 (2), Yellow No. 203, Orange No. 205, Orange No. 207, Orange No. 402, Green No. 3, Green No. 204, Green No. 401, Purple No. 401, Blue No. 1, Blue No. 2, Blue No. 202, Brown No. 201, and Black No. 401.

Examples of the basic dye may include Basic Blue 3, Basic Blue 6, Basic Blue 7, Basic Blue 9, Basic Blue 26, Basic Blue 41, Basic Blue 47, Basic Blue 99, Basic Brown 4, Basic Brown 16, Basic Brown 17, Basic Green 1, Basic Green 4, Basic Orange 1, Basic Orange 2, Basic Orange 31, Basic Red 1, Basic Red 2, Basic Red 22, Basic Red 46, Basic Red 51, Basic Red 76, Basic Red 118, Basic Violet 1, Basic Violet 3, Basic Violet 4, Basic Violet 10, Basic Violet 11: 1, Basic Violet 14, Basic Violet 16, Basic Yellow 11, Basic Yellow 28, Basic Yellow 57, and Basic Yellow 87.

Examples of the natural dye may include gardenia pigment, turmeric pigment, annatto pigment, sodium copper chlorophyllin, paprika pigment, lac pigment, and henna.

Examples of the nitro dye may include 4-nitro-o-phenylenediamine, 2-nitro-p-phenylenediamine, 2-amino-4-nitrophenol, 2-amino-5-nitrophenol, picramic acid, picric acid, and a salt thereof.

Examples of the HC dye may include HC Blue No. 2, HC Blue No. 5, HC Blue No. 6, HC Blue No. 9, HC Blue No. 10, HC Blue No. 11, HC Blue No. 12, HC Blue No.13, HC Orange No. 1, HC Orange No. 2, HC Orange No. 3, HC Red No. 1, HC Red No. 3, HC Red No. 7, HC Red No. 10, HC Red No. 11, HC Red No. 13, HC Red No. 14, HC Violet No. 1, HC Violet No. 2, HC Yellow No. 2, HC Yellow No. 4, HC Yellow No. 5, HC Yellow No. 6, HC Yellow No. 9, HC Yellow No. 10, HC Yellow No. 11, HC Yellow No. 12, HC Yellow No. 13, HC Yellow No. 14, and HC Yellow No. 15.

Examples of the disperse dye may include Disperse Black 9, Disperse Blue 1, Disperse Blue 3, Disperse Blue 7, Disperse Brown 4, Disperse Orange 3, Disperse Red 11, Disperse Red 15, Disperse Red 17, Disperse Violet 1, Disperse Violet 4, and Disperse Violet 15.

### <Oil component>

Examples of the oil component may include a higher alcohol, fats and oils, waxes, a hydrocarbon, a higher fatty acid, esters, silicone oil, and fluorine oil. One kind or two or more kinds can be selected from these oil components and used.

Examples of the higher alcohol may include cetyl alcohol (cetanol), stearyl alcohol, cetostearyl alcohol, oleyl alcohol, linoleyl alcohol, linolenyl alcohol, arachyl alcohol, behenyl alcohol, lauryl alcohol, myristyl alcohol, 2-hexyldecanol, isostearyl alcohol, 2-octyldodecanol, decyltetradecanol, phytosterol, phytostanol, cholesterol, cholestanol, lanosterol, and ergosterol.

Fats and oils are triglycerides, namely triesters of fatty acids with glycerin. Examples thereof may include olive oil, rose hip oil, camellia oil, shea butter, macadamia nut oil, almond oil, tea seed oil, sasanqua oil, safflower oil, sunflower oil, soybean oil, cottonseed oil, sesame oil, beef tallow, cacao butter, corn oil, peanut oil, rapeseed oil, rice bran oil, rice germ oil, wheat germ oil, pearl barley oil, grape seed oil, avocado oil, carrot oil, castor oil, linseed oil, coconut oil, mink oil, and egg yolk oil.

A hydrocarbon is a compound composed of carbon and hydrogen. Examples thereof may include liquid paraffin, paraffin, microcrystalline wax, petroleum jelly, isoparaffins, ozokerite, ceresin, polyethylene, α-olefin oligomer, polybutene, synthetic squalane, squalene, hydrogenated squalane, limonene, and turpentine oil.

Examples of the higher fatty acid may include lauric acid, myristic acid, palmitic acid, stearic acid, behenic acid, isostearic acid, hydroxystearic acid, 12-hydroxystearic acid, oleic acid, undecylenic acid, linoleic acid, ricinoleic acid, and lanolin fatty acid.

Esters are compounds obtained by the dehydration reaction of fatty acids with alcohols. Examples thereof may include diisopropyl adipate, 2-hexyldecyl adipate, isopropyl myristate, myristyl myristate, cetyl octanoate, cetyl isooctanoate, isononyl isononanoate, diisopropyl sebacate, isopropyl palmitate, 2-ethylhexyl palmitate, cetyl ethylhexanoate, butyl stearate, isocetyl isostearate, hexyl laurate, decyl oleate, fatty acid (C10-30) (cholesteryl/lanosteryl), lauryl lactate, octyldodecyl lactate, lanolin acetate, dipentaerythritol fatty acid ester, monoisostearic acid N-alkyl glycol, and a lanolin derivative.

A silicone oil is a synthetic polymer in which silicon to which an organic group is attached and oxygen are alternately connected to each other by a chemical bond. Examples thereof may include dimethylpolysiloxane (INCI name: dimethicone), dimethyl polysiloxane having a hydroxyl end group (INCI name: dimethiconol), methylphenyl polysiloxane, decamethylcyclopentasiloxane, dodecamethylcyclohexasiloxane, polyether-modified silicone, highly polymerized silicone having an average polymerization degree of from 650 to 10000, amino-modified silicone, betaine-modified silicone, alkyl-modified silicone, alkoxy-modified silicone, mercapto-modified silicone, carboxy-modified silicone, and fluorine-modified silicone.

Among these, examples of the amino-modified silicone may include an aminopropylmethylsiloxane-dimethylsiloxane copolymer (INCI name: aminopropyl dimethicone), an aminoethylaminopropylsiloxane-dimethylsiloxane copolymer (INCI name: amodimethicone), and an aminoethylaminopropylmethylsiloxane-dimethylsiloxane copolymer (INCI name: trimethylsilylamodimethicone).

### <Surfactant>

Examples of the surfactant may include a nonionic surfactant, a cationic surfactant, an anionic surfactant, and an amphoteric surfactant.

Incidentally, in the following description, POE represents a polyoxyethylene chain, POP represents a polyoxypropylene chain, and the number in parentheses following this indicates the number of moles added. In addition, the number in parentheses following alkyl indicates the number of carbon atoms in the fatty acid chain.

Examples of the nonionic surfactant may include a POE alkyl ether, a POE alkyl phenyl ether, a POE-POP alkyl ether, a POE sorbitan fatty acid ester, a POE mono fatty acid ester, a POE glycerin fatty acid ester, a polyglycerin fatty acid ester, a monoglycerin fatty acid ester, a sorbitan fatty acid ester, a sucrose fatty acid ester, and an alkyl polyglucoside. Specific examples of the POE alkyl ether may include POE lauryl ether, POE cetyl ether, POE stearyl ether, POE behenyl ether, POE lanolin, and POE phytosterol.

Examples of the cationic surfactant may include an alkyl quaternary ammonium salt such as a monoalkyl type quaternary ammonium salt, a dialkyl type quaternary ammonium salt, a trialkyl type quaternary ammonium salt, a benzalkonium type quaternary ammonium salt or a monoalkyl ether type quaternary ammonium salt, an amine salt such as an alkylamine salt, a fatty acid amidoamine salt, an ester-containing tertiary amine salt, or an arcobel type tertiary amine salt, a cyclic quaternary ammonium salt such as an alkyl pyridinium salt or an alkyl isoquinolinium salt, and benzethonium chloride.

The cationic surfactant is preferably an alkyl quaternary ammonium salt, more preferably a monoalkyl type quaternary ammonium salt or a dialkyl type quaternary ammonium salt, and particularly preferably a monoalkyl type quaternary ammonium salt.

Examples of the monoalkyl type quaternary ammonium salt may include lauryltrimethylammonium chloride, lauryltrimethylammonium bromide, alkyl (16,18) trimethylammonium chloride, cetyltrimethylammonium chloride, cetyltrimethylammonium bromide, cetyltrimethylammonium saccharin, stearyltrimethylammonium chloride, stearyltrimethylammonium bromide, behenyltrimethylammonium chloride, stearyltrimethylammonium saccharin, alkyl (28) trimethylammonium chloride, diPOE (2) oleylmethylammonium chloride, diPOEstearylmethylammonium chloride, POE (1) POP (25) diethylmethylammonium chloride, POP methyldiethylammonium chloride, methacryloyloxyethyltrimethylammonium chloride, and behenyltrimethylammonium methyl sulfate. The monoalkyl type quaternary ammonium salt is particularly preferably stearyltrimethylammonium chloride, alkyl (16,18) trimethylammonium chloride, and cetyltrimethylammonium chloride.

Examples of the anionic surfactant may include an alkyl ether sulfate, a POE alkyl ether sulfate, an alkyl sulfate, an alkenyl ether sulfate, an alkenyl sulfate, an olefin sulfonate, an alkane sulfonate, a saturated or unsaturated fatty acid salt, an alkyl or alkenyl ether carboxylate, an α-sulfone fatty acid salt, a N-acylamino acid type surfactant, a phosphoric acid mono- or diester type surfactant, and a sulfosuccinic acid ester. The counter ion of the anion group in these surfactants may be, for example, any of a sodium ion, a potassium ion, or triethanolamine.

More specific examples thereof may include disodium lauryl sulfosuccinate, sodium lauryl sulfate, sodium myristyl sulfate, potassium lauryl sulfate, ammonium lauryl sulfate, triethanolamine lauryl sulfate, sodium cetyl sulfate, sodium stearyl sulfate, sodium POE lauryl ether sulfate, triethanolamine POE lauryl ether sulfate, ammonium POE lauryl ether sulfate, sodium POE stearyl ether sulfate, sodium methyl stearoyl taurate, triethanolamine dodecylbenzenesulfonate, sodium tetradecene sulfonate, sodium lauryl phosphate, POE lauryl ether phosphoric acid and a salt thereof, N-lauroyl glutamic acid salts (sodium lauroyl glutamate and the like), a N-lauroylmethyl-β-alanine salt, a N-acylglycine salt, a N-acylglutamate, lauric acid and myristic acid which are a higher fatty acid, and a salt of these higher fatty acids, and one kind or two or more kinds of these can be used.

Examples of the amphoteric surfactant may include an amino acid type amphoteric surfactant and a betaine type amphoteric surfactant.

Specific examples of the amino acid type amphoteric surfactant may include a glycine type amphoteric surfactant such as sodium N-lauroyl-N'-carboxymethyl-N'-hydroxyethylethylenediamine (Na lauroamphoacetate), 2-alkyl-N-carboxymethyl-N-hydroxyethylimidazolinium betaine, sodium undecylhydroxyethyl imidazolinium betaine, alkyl diaminoethyl glycine hydrochloride, sodium N-coconut oil fatty acid acyl-N'-carboxyethyl-N'-hydroxyethylethylenediamine, disodium N-coconut oil fatty acid acyl-N'-carboxyethoxyethyl-N'-carboxyethylethylenediamine, disodium N-coconut oil fatty acid acyl-N'-carboxymethoxyethyl-N'-carboxymethylethylenediamine, sodium lauryl diaminoethyl glycine, or sodium palm oil fatty acid acyl-N-carboxyethyl-N-hydroxyethylethylenediamine; an aminopropionic acid type amphoteric surfactant such as sodium laurylaminopropionate, sodium laurylaminodipropionate, or triethanolamine lauryl aminopropionate.

Specific examples of the betaine type amphoteric surfactant may include an aminoacetic acid betaine type amphoteric surfactant such as coconut oil alkyl betaine, lauryldimethylaminoacetic acid betaine, myristyldimethylaminoacetic acid betaine, stearyl dimethyl aminoacetic acid betaine, sodium stearyl dimethyl betaine, coconut oil fatty acid amidopropyl betaine, palm oil fatty acid amidopropyl betaine, lauric acid amidopropyl betaine, ricinoleic acid amidopropyl betaine, or stearyl dihydroxyethyl betaine; and sulfobetaine type amphoteric surfactant such as lauryl hydroxy sulfobetaine.

### <Inorganic salt>

Examples of the inorganic salt may include sodium sulfate. Sodium sulfate has an action of preventing the powder hair dye composition from absorbing moisture and is suitably utilized as an excipient. By containing sodium sulfate, it is possible to suppress a decrease in fluidity caused by moisture absorption, a decrease in storage stability of the oxidation dye, and the like.

### <Plant extract>

Examples of the plant extract may include aloe extract, Scutellaria root extract, Hypericum erectum extract, licorice root extract, beefsteak plant extract, hawthorn extract, rosemary extract, turmeric extract, sea weed extract, burdock extract, ginger extract, mallow extract, tea extract, hamamelis extract, saxifraga extract, citron extract, and soapberry extract.

### [Method of using powder hair dye composition]

The method of using a powder hair dye composition of the invention includes a step of mixing the powder hair dye composition described above with a liquid medium such as water to prepare a hair dye coating liquid and a step of applying the hair dye coating liquid to the hair.

The liquid medium is preferably water such as tap water, purified water, deionized water, or distilled water or a liquid medium using water as the base. More preferably the liquid medium is water.

The mixing ratio of the powder hair dye composition to the liquid medium is that powder hair dye composition : liquid medium = preferably from 1 : 5 to 1 : 15 and more preferably from 1 : 8 to 1 : 12 in terms of mass.

The formulation of the hair dye coating liquid is preferably in a cream form, a paste form, or a gel form and more preferably in a cream form or a paste form from the viewpoint of ease of handling the hair dye coating liquid with a brush and excellent spreading and adhesive property of the hair dye coating liquid to the hair.

As a means for applying the hair dye coating liquid to the hair, the hair dye coating liquid may be applied to the hair by using an applicator such as a comb, a brush, or a brush. In addition, the hair dye coating liquid may be applied to the hair with a hand wearing a glove.

For mixing of the powder hair dye composition with the liquid medium, various kinds of preparation tools, for example, a container such as a bottle, a cup, or a tray, a stirring tool such as a stirring rod, and the like can be used. The preparation tool is appropriately selected depending on the viscosity of the mixture and the like. Among the preparation tools, it is suitable to adopt a bottle. In other words, it is possible to easily and quickly mix the respective agents by enclosing the powder hair dye composition and the liquid medium in a bottle at a predetermined ratio and shaking the bottle. Furthermore, it is more suitable to use an applicator container equipped with a bottle and an applicator which is connected to the bottle and discharges the hair dye coating liquid in the bottle. By using such an applicator container, the coating operation becomes simpler since it is possible to prepare the hair dye coating liquid in the bottle and to apply the hair dye coating liquid to the hair by using the applicator. Examples of the applicator equipped in the applicator container may include an applicator in a comb form, a brush form, a nozzle form, or the like. As an applicator, a container with comb with which it is possible to apply the hair dye coating liquid to the hair while combing the hair with the comb is preferable from the viewpoint of handiness.

The leaving time after the hair dye coating liquid being applied to the hair is not particularly limited, but it may be about from 30 to 40 minutes. After the leaving time, the hair dye coating liquid adhered to the hair is washed out. Thereafter, the hair may be properly treated using a shampoo, a conditioner, a treatment, or the like.

### EXAMPLES

Hereinafter, the invention will be specifically described with reference to Examples, but the technical scope of the invention is not limited by these Examples.

### [Preparation of powder hair dye composition]

A powder hair dye composition having the composition presented in the following Tables 1 to 3 was prepared. The powder hair dye composition obtained was subjected to the evaluation on the "temporary stability of coating operability in long-term storage", "mixing property with liquid medium", "levelness of dyeing", "fluidity", and "feel of hair at time of finishing".

### [Evaluation method]

### <Temporary stability of coating operability in long-term storage>

For each Example and Comparative Example, the powder hair dye composition immediately after being prepared (hereinafter referred to as the "sample before storage") and a powder hair dye composition stored at 50°C and a humidity of 80% for 60 days (hereinafter referred to as the "sample after storage") were prepared, and each sample was subjected to the following coating operation test.

### (Coating operation test)

Into a mixing container having a capacity of 100 mL, 3 g of the powder hair dye composition of Examples and Comparative Examples and 30 g of water were charged and mixed by using a stirring rod to prepare a hair dye coating liquid. In the operation of applying each hair dye coating liquid to the hair of head of human by using a brush, the operability was evaluated according to the following criteria.

The results for the sample before storage and the sample after storage are presented as the items of "coating operability (before storage)" and "coating operability (after storage)" in Tables 1 to 3, respectively.
5: It is significantly easy to handle hair dye coating liquid with brush and spreading and adhesive property of hair dye coating liquid to hair of head are favorable.
4: It is easy to handle hair dye coating liquid with brush and spreading and adhesive property of hair dye coating liquid to hair of head are favorable.
3: It is possible to handle hair dye coating liquid with brush and spreading and adhesive property of hair dye coating liquid to hair of head are favorable.
2: It is difficult to handle hair dye coating liquid with brush.
1: It is difficult to handle hair dye coating liquid with brush and spreading and adhesive property of hair dye coating liquid to hair of head are insufficient.
-: Viscosity is low and hair dye coating liquid fall in drop from brush.

### <Mixing property with liquid medium>

Into a mixing container (diameter: 100 mm, cylindrical shape) having a flat bottom, 6 g of the powder hair dye composition of each Example and Comparative Example was charged and levelled off. Thereinto, 60 g of water was charged, these were mixed together by using a brush, and the mixture was mixed so as to draw a circle for 30 seconds. Thereafter, the mixture was mixed so as to crush the lump with the brush, and the time taken from the start of mixing to completion of mixing was measured. The mixing property with a liquid medium was evaluated according to the following criteria. The results are presented as the item of "mixing property" in Tables 1 to 3.
5: Powder hair dye composition is completely mixed with liquid medium in 1 minute.
4: Powder hair dye composition is completely mixed with liquid medium after 1 minute but in 1 minute 20 seconds.
3: Powder hair dye composition is completely mixed with liquid medium after 1 minute 20 seconds but in 1 minute 40 seconds.
2: Powder hair dye composition is completely mixed with liquid medium after 1 minute 40 seconds but in 2 minutes.
1: It takes 2 minutes or longer to completely mix powder hair dye composition with liquid medium.

### <Levelness of dyeing>

Using the powder hair dye composition of each Example and Comparative Example, a white hair bundle was dyed, and the levelness of dyeing of the dyed hair bundle thus obtained was visually evaluated. Incidentally, the hair dyeing treatment of the white hair bundle was performed by the following method of hair dyeing treatment.

### (Method of hair dyeing treatment)

Into a mixing container having a capacity of 100 mL, 3 g of the powder hair dye composition of each Example and Comparative Example and 30 g of water were charged and mixed by using a stirring rod to prepare a hair dye coating liquid. The hair dyeing treatment was performed by applying 3 g of the hair dye coating liquid obtained to 1 g of white hair bundle by using a brush and leaving the hair dye coating liquid for 40 minutes after the coating operation. The hair bundle subjected to the hair dyeing treatment was washed with water and a shampoo to wash out the hair dye coating liquid from the hair bundle, and treated with a conditioner, then the water was wiped off from the hair bundle with a towel, and the hair bundle was dried by using a dryer.

### (Evaluation on levelness of dyeing)

Next, the degree of color unevenness of the dyed hair bundle thus obtained was visually observed by ten panelists to evaluate (sensory evaluation) the presence or absence of color unevenness. Specifically, it was judged as "5" in a case in which the "hair bundle is dyed without unevenness", as "4" in a case in which the "hair bundle is dyed almost without unevenness", as "3" in a case in which the "hair bundle is dyed without severe unevenness", as "2" in a case in which the "unevenness is severe", and as "1" in a case in which the "unevenness is significantly severe". The average score of the evaluation results by the ten panelists thus obtained was calculated and taken as the evaluation results on the levelness of dyeing. Incidentally, the numerical values after the decimal point were rounded off.

The results are presented as the item of "levelness of dyeing" in Tables 1 to 3.

### <Fluidity>

Into a transparent hermetically sealed container having a capacity of 100 mL, 20 g of the powder hair dye composition of each Example and Comparative Example was charged and the hermetically sealed container was shaken to examine the fluidity of the powder. The fluidity was evaluated based on the evaluation result of the powder hair dye composition of Example 1, and it was judged as "○" for those having fluidity equal to or higher than that of Example 1, as "Δ" for those having fluidity slightly lower than that of Example 1, and as "×" for those having fluidity extremely lower than that of Example 1. The results are presented as the item of "fluidity" in Tables 1 to 3.

### <Feel of hair at time of finishing>

The hair bundle subjected to the hair dyeing treatment in the evaluation on the "levelness of dyeing" described above was washed with a commercially available shampoo ("Bigen Treatment Shampoo" manufactured by Hoyu Co., Ltd.) and a commercially available rinse ("Bigen Treatment Rinse" manufactured by Hoyu Co., Ltd.) and then dried by using a drier.

The feel of the hair at the time of finishing was evaluated to be "significantly excellent: 5" in a case in which the number of panelists who evaluated the stiffness of the hair-bundle sample to be favorable in two-rank evaluation of favorable and poor was 9 or more among ten expert panelists, to be "excellent: 4" in a case in which the number of panelists was 7 to 8, to be "favorable: 3" in a case in which the number of panelists was 5 to 6, to be "slightly poor: 2" in a case in which the number of panelists was 3 to 4, and to be "poor: 1" in a case in which the number of panelists was 2 or fewer.

The results are presented as the item of "feel (hair stiffness)" in Tables 1, 2, and 3.

### [Table 1]

**Table 1**

| (Unit: mass%) | | | | | | | | |
|---|---|---|---|---|---|---|---|---|
| | | | Example 1 | Example 2 | Comparative Example 1 | Comparative Example 2 | Comparative Example 3 | Comparative Example 4 |
| (A) | | sodium percarbonate | 20 | 20 | 20 | 20 | 20 | 20 |
| (B) | b1 | Xanthan gum | 15 | - | 15 | 15 | 15 | - |
| | | Chitosan | - | 15 | - | - | - | - |
| | b2 | Starch | 25 | 25 | - | - | - | 25 |
| | | Sodium carboxymethyl cellulose | - | - | - | 10 | 25 | - |
| (C) | | Magnesium stearate | 1 | 1 | 1 | 1 | 1 | 1 |
| (D) | | Ethylenediamine hydroxyethyl triacetic acid trisodium dihydrate | 2 | 2 | 2 | 2 | 2 | 2 |
| Disodium lauryl sulfosuccinate | | | 1 | 1 | 1 | 1 | 1 | 1 |
| Sodium sulfate | | | 13 | 13 | 38 | 28 | 13 | 28 |
| p-Phenylenediamine sulfate | | | 10 | 10 | 10 | 10 | 10 | 10 |
| Toluene-2,5-diamine sulfate | | | 5 | 5 | 5 | 5 | 5 | 5 |
| m-Aminophenol sulfate | | | 2 | 2 | 2 | 2 | 2 | 2 |
| p-Aminophenol sulfate | | | 2 | 2 | 2 | 2 | 2 | 2 |
| 5-Amino-o-cresol sulfate | | | 2 | 2 | 2 | 2 | 2 | 2 |
| p-Methylaminophenol sulfate | | | 2 | 2 | 2 | 2 | 2 | 2 |
| Sum | | | 100 | 100 | 100 | 100 | 100 | 100 |
| b2/b1 | | | 1.67 | 1.67 | - | - | - | - |
| b2/D | | | 12.50 | 12.50 | - | - | - | 12.50 |
| Evaluation | | Coating operability (before storage) | 5 | 5 | 4 | 5 | 3 | - |
| | | Coating operability (after storage) | 5 | 4 | 3 | 2 | 2 | - |
| | | Mixing property | 5 | 5 | 5 | 5 | 1 | 5 |
| | | Levelness of dyeing | 5 | 4 | 2 | 2 | 2 | 2 |
| | | Fluidity | ○ | ○ | ○ | ○ | ○ | ○ |
| | | Feel (hair stiffness) | 5 | 4 | 2 | 3 | 3 | 3 |

Referring to Table 1, an excellent effect is acknowledged in all the evaluation items in the case of the powder hair dye compositions of Examples 1 and 2.

Next, when Example 1 is compared with Comparative Examples 1 and 4 in Table 1 in order to investigate the action of each component in detail, it is acknowledged that deterioration is suppressed in the coating operability in long-term storage and also the hair stiffness at the time of finishing is ameliorated by concurrently using xanthan gum which is the component b1 with starch which is the component b2. In addition, it is acknowledged that the levelness of dyeing is ameliorated by containing a starch since the levelness of dyeing is evaluated to be high in both of Example 1 and Comparative Example 4.

Incidentally, referring to Comparative Examples 2 and 3, degradation in the coating operability in long-term storage is acknowledged in a case in which sodium carboxymethyl cellulose to be utilized in the conventional powder hair dye composition is blended instead of a starch. In addition, the "levelness of dyeing" and "feel of hair at time of finishing " are both evaluated to be low. In other words, it can be said that the powder hair dye composition of the invention exerts an exceptional and remarkable effect as compared with the conventional powder hair dye composition by concurrently using the component b1 with the component b2.

### [Table 2]

**Table 2**

| (Unit: mass%) | | | | | | | |
|---|---|---|---|---|---|---|---|
| | | | Example 1 | Example 3 | Example 4 | Example 5 | Example 6 |
| (A) | | Sodium percarbonate | 20 | 20 | 20 | 20 | 20 |
| (B) | b1 | Xanthan gum | 15 | 7 | 20 | 15 | 15 |
| | b2 | Starch | 25 | 25 | 25 | 5 | 38 |
| (C) | | Magnesium stearate | 1 | 1 | 1 | 1 | 1 |
| (D) | | Ethylenediamine hydroxyethyl triacetic acid trisodium dihydrate | 2 | 2 | 2 | 2 | 2 |
| Disodium lauryl sulfosuccinate | | | 1 | 1 | 1 | 1 | 1 |
| Sodium sulfate | | | 13 | 21 | 8 | 33 | - |
| p-Phenylenediamine sulfate | | | 10 | 10 | 10 | 10 | 10 |
| Toluene-2,5-diamine sulfate | | | 5 | 5 | 5 | 5 | 5 |
| m-Aminophenol sulfate | | | 2 | 2 | 2 | 2 | 2 |
| p-Aminophenol sulfate | | | 2 | 2 | 2 | 2 | 2 |
| 5-Amino-o-cresol sulfate | | | 2 | 2 | 2 | 2 | 2 |
| p-Methylaminophenol sulfate | | | 2 | 2 | 2 | 2 | 2 |
| Sum | | | 100 | 100 | 100 | 100 | 100 |
| b2/b1 | | | 1.67 | 3.57 | 1.25 | 0.33 | 2.53 |
| b2/D | | | 12.50 | 12.50 | 12.50 | 2.50 | 19.00 |
| Evaluation | | Coating operability (before storage) | 5 | 5 | 4 | 5 | 4 |
| | | Coating operability (after storage) | 5 | 5 | 4 | 4 | 4 |
| | | Mixing property | 5 | 5 | 5 | 5 | 5 |
| | | Levelness of dyeing | 5 | 5 | 5 | 3 | 4 |
| | | Fluidity | ○ | ○ | ○ | ○ | Δ |
| | | Feel (hair stiffness) | 5 | 5 | 4 | 4 | 5 |

Referring to Examples 3 and 4 in Table 2, it can be seen that sufficient coating operability is obtained when the content of the component b1 is in a range of from 7 to 20 mass%. In addition, referring to Examples 5 and 6, it can be seen that the effect of the invention is further exerted when the content of the component b2 is in a range of 5 mass% or more and the evaluation result on the "fluidity" is improved when the content of the component b2 is lower than 38 mass%.

Next, as the effect obtained by concurrently using the component b1 with the component b2, deterioration is suppressed in the coating operability in long-term storage, and it can be seen that an excellent effect is exerted by setting the ratio (b2/b1) of the content of the component b1 to the content of the component b2 to 0.33 or more when improvement in the feel of the hair at the time of finishing is further investigated.

### [Table 3]

**Table 3**

| (Unit: mass%) | | | | | | | |
|---|---|---|---|---|---|---|---|
| | | | Example 1 | Example 7 | Example 8 | Example 9 | Example 10 |
| (A) | | Sodium percarbonate | 20 | 15 | 33 | 20 | 20 |
| (B) | b1 | Xanthan gum | 15 | 15 | 15 | 15 | 15 |
| | b2 | Starch | 25 | 25 | 25 | 25 | 25 |
| (C) | | Magnesium stearate | 1 | 1 | 1 | 0.1 | 2 |
| (D) | | Ethylenediamine hydroxyethyl triacetic acid trisodium dihydrate | 2 | 2 | 2 | 2 | 2 |
| Disodium lauryl sulfosuccinate | | | 1 | 1 | 1 | 1 | 1 |
| Sodium sulfate | | | 13 | 18 | - | 13.9 | 12 |
| p-Phenylenediamine sulfate | | | 10 | 10 | 10 | 10 | 10 |
| Toluene-2,5-diamine sulfate | | | 5 | 5 | 5 | 5 | 5 |
| m-Aminophenol sulfate | | | 2 | 2 | 2 | 2 | 2 |
| p-Aminophenol sulfate | | | 2 | 2 | 2 | 2 | 2 |
| 5-Amino-o-cresol sulfate | | | 2 | 2 | 2 | 2 | 2 |
| p-Methylaminophenol sulfate | | | 2 | 2 | 2 | 2 | 2 |
| Sum | | | 100 | 100 | 100 | 100 | 100 |
| b2/b1 | | | 1.67 | 1.67 | 1.67 | 1.67 | 1.67 |
| b2/D | | | 12.50 | 12.50 | 12.50 | 12.50 | 12.50 |
| Evaluation | | Coating operability (before storage) | 5 | 5 | 5 | 5 | 5 |
| | | Coating operability (after storage) | 5 | 5 | 5 | 5 | 5 |
| | | Mixing property | 5 | 5 | 5 | 4 | 3 |
| | | Levelness of dyeing | 5 | 4 | 5 | 5 | 5 |
| | | Fluidity | ○ | ○ | ○ | Δ | ○ |
| | | Feel (hair stiffness) | 5 | 5 | 5 | 5 | 5 |

Referring to Examples 7 and 8 in Table 3, it can be seen that the preferred range of the content of the component A is 15 mass% or more.

In addition, referring to Examples 9 and 10, extremely excellent effects are acknowledged in the evaluation on the "mixing property with liquid medium" and "fluidity" by setting the content of magnesium stearate which is the component C to from 0.1 to 2 mass%.

### INDUSTRIAL APPLICABILITY

The powder hair dye composition of the invention can be utilized as a powder hair dye composition for dyeing body hair such as hair of head, beard, eyebrows, and leg hair of human. In addition to this, the powder hair dye composition of the invention may be utilized for dyeing body hair of an animal such as a pet.

The powder hair dye composition of the invention can be utilized as a powder hair dye composition for beauty parlor or barber shop and a powder hair dye composition for self coloring.

In addition, the method of using a powder hair dye composition of the invention can be utilized for hair dyeing treatment of hair in a beauty parlor, a barber shop or the like and for self coloring.

## Claims

1. A powder hair dye composition comprising (A) a percarbonate and (B) a water-soluble polymer compound, wherein
(B) the water-soluble polymer compound contains (b1) a microorganism-derived/produced mucilage or chitosan containing glucose as a constitutive sugar and (b2) a starch.

2. The powder hair dye composition according to claim 1, wherein a mass ratio (b2/b1) of (b2) the starch to (b1) the microorganism-derived/produced mucilage or chitosan containing glucose as a constitutive sugar is from 0.1 to 5.

3. The powder hair dye composition according to claim 1 or 2, further comprising (C) a metal salt of stearic acid at from 0.1 to 2 mass%.

4. The powder hair dye composition according to claim 1 or 2, further comprising (D) a chelating agent at from 1 to 5 mass%.

5. The powder hair dye composition according to claim 4, wherein (b1) is xanthan gum,
a content of the xanthan gum is from 8 to 18 mass% and a content of (b2) the starch is from 7 to 35 mass%, and
a mass ratio (b2/D) of (b2) the starch to (D) the chelating agent is 3.5 or more.

6. A method of using a powder hair dye composition, comprising:
a step of mixing the powder hair dye composition according to claim 1 or 2 with a liquid medium to prepare a hair dye coating liquid; and
a step of applying the hair dye coating liquid to hair.
